(19) Europäisches Patentamt
European Patent Office
Office européen des brevets

(11) Veröffentlichungsnummer: **0 190 102**
**B1**

(12) # EUROPÄISCHE PATENTSCHRIFT

(45) Veröffentlichungstag der Patentschrift: **25.04.90**

(51) Int. Cl.$^5$: **C 07 D 209/76,** C 08 F 12/32

(21) Anmeldenummer: **86810039.7**

(22) Anmeldetag: **23.01.86**

(54) **Substituierte ungesättigte, sulfonyloxygruppenhaltige bicyclische Imide als Katalysatoren für die kationische Polymerisation.**

(30) Priorität: **29.01.85 CH 388/85**

(43) Veröffentlichungstag der Anmeldung:
**06.08.86 Patentblatt 86/32**

(45) Bekanntmachung des Hinweises auf die Patenterteilung:
**25.04.90 Patentblatt 90/17**

(84) Benannte Vertragsstaaten:
**BE CH DE FR GB IT LI NL**

(56) Entgegenhaltungen:
**EP-A-0 105 024**
**EP-A-0 152 372**
**EP-A-0 166 693**
**US-A-3 450 711**

(73) Patentinhaber: **CIBA-GEIGY AG**
**Klybeckstrasse 141**
**CH-4002 Basel (CH)**

(72) Erfinder: **Renner, Alfred, Dr.**
**Marcoup 2**
**CH-3280 Muntelier (CH)**
Erfinder: **Vonlanthen, Christian**
**Le Saugy**
**CH-1751 Lentigny (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft mit Allyl oder Methallyl und gegebenenfalls Methyl substituierte sulfonyloxygruppenhaltige Bicyclo[2.2.1]hept-5-en-2,3-dicarbonsäureimide, deren Herstellung und deren Verwendung als Katalysatoren für die kationische Polymerisation von bestimmten ungesättigten Imiden.

Das US Patent 3,450,711 beschreibt Bisimidverbindungen, hergestellt durch Umsetzung von Endo, cis-bicyclo[2.2.1]hept-5-en-2,3-dicarbonsäureanhydrid (= 5-Norbornen-2,3-dicarbonsäureanhydrid) mit ausgewählten organischen Diaminen. Diese Bisimide enthalten weder Methylnoch Methallyl- oder Allylsubstituenten im Imidrest und unterscheiden sich von den vorliegenden Verbindungen sowohl durch ihre Struktur als auch durch ihre chemische Reaktivität. Die Verbindungen gemäss diesem US Patent werden als Zwischenprodukte bei der Herstellung von Epoxidverbindungen verwendet.

Die EP-Patentanmeldung 0 105 024 beschriebt allyl- oder methallyl-substituierte Bicyclo[2.2.1]hept-5-en-2,3-dicarbonsäureimide und die daraus erhältlichen Polymeren.

Die erfindungsgemässen sulfonyloxygruppenhaltigen Bicyclo[2.2.1]hept-5-en-2,3-dicarbonsäureimide sind wertvolle latente Katalysatoren für die kationische Polymerisation von bestimmten ungesättigten Imiden, wodurch vernetzte Polymere mit ausgezeichneten Eigenschaften erhältlich sind. Die Imide sind durch die folgende Formel I gekennzeichnet:

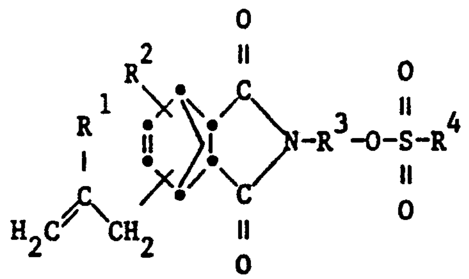

(I),

worin $R^1$ und $R^2$ unabhängig voneinander Wasserstoff oder Methyl bedeuten, $R^3$ eine direkte Bindung oder ein gegebenenfalls durch O-Atome in der Kette unter brochener $C_2$—$C_{20}$-aliphatischer, ein ein- oder mehrkerniger $C_5$—$C_{20}$-cycloaliphatischer oder ein $C_6$—$C_{20}$-aromatischer Rest ist oder für eine Gruppe der Formel II steht

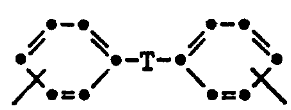

(II),

worin T Methylen, Isopropyliden, CO, O, S oder $SO_2$ bedeutet, und $R^4$ für $C_1$—$C_{12}$-Alkyl, $C_5$—$C_8$-Cycloalkyl, $C_6$—$C_{10}$-Aryl, Benzyl oder $C_7$—$C_{12}$-Alkaryl steht.

Vorzugsweise bedeuten $R^1$ und $R^2$ je ein Wasserstoffatom.

$R^3$ kann ein zweiwertiger geradkettiger oder verzweigter aliphatischer Rest mit 2—20, vorzugsweise 2—12 und insbesondere 2—6 C-Atomen sein, der in der Kette durch ein oder mehrere Sauerstoffatome unterbrochen sein kann. Beispiele für geeignete aliphatische Reste $R^3$ sind Ethylen, 1,2- und 1,3-Propylen, Butylen, Penta- und Hexamethylen, Heptylen, Octylen, Decylen, Dodecylen, Hexadecylen und Neopentylen. Durch Sauerstoffatome unterbrochene aliphatische Reste können beispielsweise von Ethylenglykol oder Propylenglykol abgeleitet sein und können Gruppen der Formeln $-CH_2CH_2\text{-}[OCH_2CH_2]_m\text{-}$ oder $-CH_2CH_2CH_2\text{-}[OCH_2CH_2CH_2]_m\text{-}$ mit m = 1—15 entsprechen.

$R^3$ kann auch ein ein- oder mehrkerniger cycloaliphatischer zweiwertiger Rest mit 5—20 C-Atomen sein, wie beispielsweise Cyclopentylen, Cyclohexylen, Cycloheptylen, Cyclooctylen, Bis(cyclohexylen)-methan, 2,2-Bis(cyclohexylen)propan und Decalinylen.

Wenn $R^3$ einen aromatischen Rest bedeutet, so handelt es sich vorzugsweise um 1,3- oder 1,4-Phenylen oder Naphthylen, die jeweils, falls gewünscht, auch durch eine oder mehrer $C_{1-4}$-Alkylgruppen, wie Methyl, Ethyl oder Propyl, substituiert sein können. Bevorzugt sind die genannten Gruppen unsubstituiert. 1,3- und 1,4-Phenylengruppen sind als aromatische Reste besonders bevorzugt.

Wenn $R^3$ eine Gruppe der Formel II bedeutet, steht T vorzugsweise für O, $SO_2$ und insbesondere für Methylen oder Isopropyliden.

Besonders bevorzugte Verbindungen der Formel I sind solche, worin $R^3$ eine direkte Bindung, eine $C_2$—$C_{10}$-Aldiphatischer Rest oder eine Gruppe $-CH_2CH_2\text{-}[OCH_2CH_2]_m\text{-}$ mit m = 1 oder 2, ein $C_5$—$C_6$-cycloaliphatischer oder ein $C_6$—$C_{10}$-aromatischer Rest ist oder für eine Gruppe der Formel II steht, worin T Methylen oder Isopropyliden ist.

$R^4$ kann eine verzweigte oder vorzugsweise geradkettige Alkylgruppe mit 1—12, vorzugsweise 1—6 und insbesondere 1 oder 2 C-Atomen sein. Beispiels für geeignete Alkylgruppen sind Dodecyl, Decyl, Octyl, Heptyl, Butyl, Propyl und insbesondere Ethyl oder Methyl.

Wenn $R^4$ einen Cycloalkylrest bedeutet, so handelt es sich vorzugsweise um Cyclopentyl oder

Cyclohexyl, die jeweils durch eine oder mehrere $C_{1-3}$-Alkylgruppen substituiert sein können. Bevorzugt sind die genannten Gruppen unsubstituiert.

Wenn $R^4$ einen Arylrest bedeutet, so handelt es sich vorzugsweise um Phenyl oder Naphthyl. Als $C_7$—$C_{12}$-Alkaryl kommen z.B. durch eine oder mehrere $C_1$—$C_3$-Alkylgruppen substituiertes Phenyl oder Naphthyl in Frage vorzugsweise Tolyl.

Besonders bevorzugte Verbindungen der Formel I sind solche, worin $R^4$ für $C_1$—$C_6$-Alkyl, $C_5$—$C_6$-Cycloalkyl, $C_6$—$C_{10}$-Aryl oder $C_7$—$C_{12}$-Alkaryl steht.

Ganz besonders bevorzugt sind Verbindungen der Formel I, worin $R^3$ eine direkte Bindung oder den Rest

$-CH_2CH_2-$,

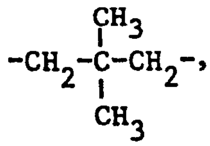, $-CH_2CH_2OCH_2CH_2-$ oder 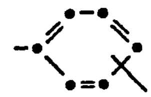 und

$R^4$ Methyl, Phenyl, 2-Naphthyl oder p-Tolyl bedeuten.

Am meisten bevorzugt sind die Verbindungen der Formel I, worin $R^1$ und $R^2$ je Wasserstoff bedeuten, $R^3$ für eine direkte Bindung oder für den Rest —$CH_2CH_2$— und $R^4$ für Methyl oder Phenyl stehen.

Die erfindungsgemässen Imide Können auf an sich bekannte Weise hergestellt werden, z.B. durch Umsetzen eines Anhydrids der Formel III

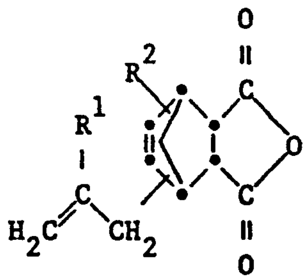

(III)

mit einer Verbindung der Formel IV

$$H_2N—R^3—OH \qquad (IV)$$

bei erhöhter Temperatur und unter Abdestillieren des bei der Reaktion entstehenden Wassers zu einer Verbindung der Formel V

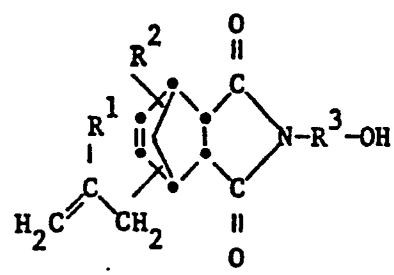

(V)

und durch anschliessendes Umsetzen des Imids V mit einem Sulfonsäurechlorid der Formel VI

$$R^4—SO_2Cl \qquad (VI)$$

unter Kühlung und ein Gegenwart eines HCl-Akzeptors, vorzugsweise eines Amins, wie z.B. Triethylamin, wobei $R^1$, $R^2$, $R^3$ und $R^4$ die unter Formel I angegebene Bedeutung haben.

Sofern es sich bei den Verbindungen der Formel IV um niedrigsiedende Amine handelt, ist ein Ueberschuss dieser Reaktanden zu empfehlen. Die Umsetzung kann ohen Lösungsmittel oder in Gegenwart eines inerten Lösungsmittels, das für die azeotrope Entfernung des Wassers verwendbar ist (Schleppmittel) erfolgen. Die Temperatur der Umsetzung kann zwischen 100 und 250°C liegen. Bevorzugt werden die Imide der Formel I in der Schmelze bei einem Druck von höchstens 4500 Pa bei Temperaturen zwischen 130 und 220°C, insbesondere 180 und 220°C hergestellt.

Die Ausgangsstoffe der Formel III können gemäss dem in der US-Patentschrift 3,105,839 beschriebenen Verfahren hergestellt werden, indem man Natrium-Cyclopentadienid oder Natriummethylcyclopentadienid mit einem Allyl- oder Methallylhalogenid umsetzt, worauf sich eine Diels-Alder-Reaktion mit Maleinsäureanhydrid anschliesst. Obgleich in der US Patentschrift angegeben wird, dass die

Allylgruppe in 7-Stellung des bicyclischen Systems gebunden ist, zeigen neuere Untersuchungen, dass eine isomere Mischung in Bezug auf die Stellung der Allylgruppe (in 1 und 6 Position) und auch auf die Endo- und Exokonfiguration des Anhydridteils gebildet wird.

Verbindungen der Formel IV sind bekannt oder können nach an sich bekannten Verfahren hergestellt werden, genauso wie Verbindungen der Formel VI.

Die Umsetzung von Verbindugnen der Formel V mit den Sulfonsäurechloriden der Formel VI erfolgt vorzugsweise mit einem äquimolaren Verhältnis der Reaktanden in einem inerten Lösungsmittel mit einem Siedepunkt unter 200°C. Als Lösungsmittel kommen beispielsweise aliphatische oder alicyclische, gegebenenfalls chlorierte Kohlenwasserstoffe und insbesondere aromatische, gegebenenfalls chlorierte Kohlenwasserstoffe, wie z.B. Chlorbenzol, Xylol und insbesondere Toluol in Betracht. Um die während der Reaktion entstehende Salzsäure zu binden, wird die Umsetzung in Gegenwart eines HCl-Akzeptors, vorzugsweise eines tertiären Amins, wie z.B. Triethylamin, Dimethylanilin, Pyridin oder Lutidin, durchgeführt. Da die Reaktion exotherm verläuft, wird mit Kühlung gearbeitet, so dass die Temperatur des Reaktionsgemisches vorzugsweise 10°C nicht übersteigt.

Die erfindungsgemässen Verbindungen sind flüssige oder neidrigschmelzende feste Stoffe. Bei erhöhter Temperatur setzen die vorliegenden sulfonyloxygruppen enthaltenden Imide die entsprechende Sulfonsäure, $R^4SO_3H$, frei, wobei $R^4$ die unter Formel I angegebene Bedeutung hat, und eignen sich dadurch als latente Katalysatoren für die Vernetzung von bestimmten kationisch polymerisierbaren ungesättigten Imiden. Der nach der Abspaltung der Sulfonsäure verbliebene Rest der erfindungsgemässen Verbindungen wird dank seiner Polyfunktionalität in das Endpolymere mit eingebaut. Es kann auch ein Gemisch von mehreren der oben beschriebenen Verbindungen der Formel I erfindungsgemäss als Katalysator eingesetzt werden.

Ein weiterer Gegenstand der vorliegenden Erfindung sind härtbare Gemische enthaltend (a) ein oder mehrere Imide der Formel I und b) ein Alkenylnadicimid.

Für die erfindungsgemässen härtbaren Zusammensetzungen geeignete Alkenylnadicimide sind beispielsweise solche, die in der EP-Patentanmeldung 0105 024 beschrieben sind, wobei diese für sich allein oder als Gemische von mindestens zwei Komponenten eingesetzt werden können:

Die bei der vorliegenden Erfindung am meisten bevorzugten Alkenylnadicimide sind die oben erwähnten substituierten Bicyclo[2.2.1]hept-5-en-2,3-dicarbonsäureimide gemäss der EP—A—0 105 024. Sie können im Bicycloheptenring auch noch eine Methylgruppe enthalten.

Die Konzentration des gemäss der vorliegenden Erfindung zu verwendenden Katalysators der Formel I liegt zweckmässig zwischen 0,1 und 15,0, vorzugsweise zwischen 0,25 und 5,0 und besonders bevorzugt zwischen 0,5 und 2,0 Gew.-%, bezogen auf das kationisch polymerisierbare Material.

Die Umsetzung erfolgt bevorzugt in der Schmelze oder teilweise in der Schmelze und teilweise in der festen Phase. Sie kann aber auch in Lösung durchgeführt werden. In den meisten Fällen erübrigt sich jedoch ein Zusatz von Lösungsmitteln, weil die Ausgangsgemische als solche bereits flüssige oder niedrigschmelzende feste Stoffe sind.

Als Lösungsmittel eignen sich z.B. aromatische Kohlenwasserstoffe wie Benzol, Toluol, und Xylol, ferner Tetralin und Decalin, sowie chlorierte Kohlenwasserstoffe wie Ethylenchlorid und Chlorbenzol sowie Glykolether wei z.B. der Monomethyl, -ethyl, -butyl und -phenylether von Ethylen- und Diethylenglykol.

Das Verfahren kann in folgender Weise auch zweistufig ausgeführt werden. Nach dem Mischen und gegebenenfalls nach einer anschliessenden Vermahlung aller Ausgangsprodukte werden das Pulver oder die Flüssigkeit zunächst eine begrenzte Zeit lang vorzugsweise auf 120—170°C erhitzt. Es entsteht ein noch thermisch verformbares, teilweise lösliches Produkt. Dieses Präpolymer muss gegebenenfalls wieder zu einem verarbeitbaren Pulver vermahlen werden, bevor es bei der Endverarbeitung endgültig gehärtet wird. Die Präpolymerisation kann auch durch Erhitzen einer Lösung oder Suspension der Ausgangsmaterialien erfolgen.

Die erfindungsgemässe Herstellung der vernetzten Polymeren erfolgt in der Regel unter gleichzeitiger Formgebung zu Formkörpern, Flächengebilden, Laminaten, Verklebungen, Schaumstoffen. Dabie können den härtbaren Massen die in der Technologie der härtbaren Kunstoffe gebräuchlichen Zusätze wie Füllstoffe, Weichmacher, Pigmente, Farbstoffe, Formtrennmittel, flammhemmende Stoffe zugesetzt werden. Als Füllstoffe können z.B. Glasfasern, Kohlenstoffasern, Glimmer, Graphit, Quarzmehl, Kaolin, kolloidales Silizium-dioxid oder Metallpulver verwendet werden, als Formtrennmittel können z.B. Silkonöl, verschiedene Wachse, Zink- oder Calciumstearat usw. dienen.

Die Formgebung der nach dem erfindungsgemässen Verfahren herstellbaren Produkte kann in manchen Fällen nach dem Giessverfahren unter Anwendung einer Giessform erfolgen.

Die Formgebung kann aber auch nach dem Heisspressverfahren unter Anwendung einer Presse durchgeführt werden. Meistens genügt es, dass man nur kurz auf Temperaturen von 160 bis 250°C bei einem Druck von $9,81 \cdot 10^4$ bis $1,96 \cdot 10^7$ Pa erhitzt, und den so erhaltenen Formling ausserhalb der Presse vollständig aushärtet.

Dank der Verwendung der erfindungsgemässen Verbindungen der Formel I als Katalysatoren für die kationische Polymerisation ist eine Härtung bei tieferen Temperaturen und mit wesentlich kürzeren Zykluszeiten möglich. Die erhaltenen Produkte zeichnen sich durch vorzügliche mechanische, wie auch thermische Eigenschaften aus.

In den folgenden Beispielen wird die Herstellung einiger erfindungsgemässer Imide, deren Eigenschaften und Anwendung illustriert.

Beispiel 1:
Allylbicyclo[2.2.1]hept-5-en-2,3-dicarbonsäure-N-benzolsulfonyloxyimid

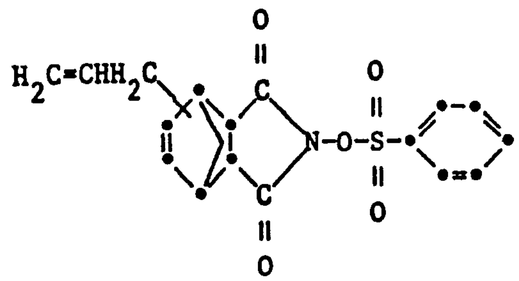

a) Allylbicyclo[2.2.1hept-5-en-2,3-dicarbonsäure-N-hydroxyimid.

Man löst 139 g (2 mol) Hydroxylaminhydrochlorid in 200 ml Wasser, fügt 408 g (2 mol) Allylbicyclo[2.2.1]hept-5-en-2,3-dicarbonsäureanhydrid (hergestellt gemäss Beispiel 1 der US Patentschrift 3 105 839) zu und tropft unter kräftigem Rühren 160 g 50%ige wässrige Natronlauge ein. Danach erhitzt man 1 Stunde auf Rückfluss, destilliert das Wasser und Spuren öliger Bestandteile ab, nimmt den Rückstand in Toluol auf, filtriert vom Kochsalz ab und entfernt das Toluol am Rotationsverdampfer bei 150°C und 2000 Pa. Es hinterbleiben 402,6 g des Produkts (91,5% d.Th.) als hellbraune viskose Flüssigkeit mit einer Viskosität von 1,28 Pa.s bei 80°C.

| Analyse: | ber. für $C_{12}H_{13}NO_3$ | gef.: |
|---|---|---|
| %C: | 65,74 | 65,34 |
| %H: | 5,98 | 6,02 |
| %N: | 6,39 | 6,40 |

b) Man löst das gemäss (a) erhaltene Produkt in 1 l Toluol und gibt 222,8 g Triethylamin zu, rührt bis eine homogene Lösung erhalten wird und kühlt auf 0°C. Man tropft unter starkem Rühren und unter äusserer Kühlung 324,7 g Benzolsulfonylchlorid so ein, dass die Temperatur des Reaktionsgemisches zwischen 5 und 10°C verbleibt. Man rührt über Nacht bei Raumtemperatur, versetzt mit Wasser, stellt mit konzentrierter Salzsäure auf pH = 5 ein und wäscht noch 2 mal mit Wasser von 75°C. Nach der Abtrennung der wässrigen Phasen trocknet man über $Na_2SO_4$, filtriert und dampft am Rotationsverdampfer bei 110°C und 2000 Pa ein. Man erhält 471 g einer viskosen Flüssigkeit, die beim Stehen kristallisiert (Fp. = 97—99°C)

| Elementaranalyse: | ber. für $C_{18}H_{17}NO_5S$ | gef.: |
|---|---|---|
| %C: | 60,16 | 60,35 |
| %H: | 4,77 | 4,85 |
| %N: | 3,90 | 3,90 |
| %S: | 8,92 | 8,59. |

IR-Spektrum ($cm^{-1}$): 575,4, 685,5 und 736,2 Aryl; 1195 und 1398 —$SO_2O$—; 1620 cycl. Doppelbindung; 1640 Allyldoppelbindung; 1742 Carbonyl.

Beispiel 2:
Methallylmethylbicyclo[2.2.1]hept-5-en-2,3-dicarbonsäure-N-benzosulfonyloxyimid

58 g Methallylmethylmethylbicyclo[2.2.1]hept-en-2,3-dicarbonsäureanhydrid (Kp = 117—120°C bei 2,7 Pa, $n_{25}^{D}$ = 1,5071;) (hergestellt analog den Beispielen 1 und 2 der US Patentschrift 3,105,839)
17,35 g Hydroxylamin-hydrochlorid,
20,78 g wässriges NaOH (48,11 Gew.%),
50 g $H_2O$ und
100 g Toluol

werden umgesetzt, wie dies im Beispiel 1a beschrieben ist. Man erhält 54,95 g eines Isomerengemisches von Methallylmethylbicyclo[2.2.1]hept-en-2,3-dicarbonsäure-N-hydroxylimid, das laut GC aus 12 Isomeren besteht.

24,7 g dieses Produkts und 11,13 g Triethylamin werden in 80 g Toluol gelöst und zwischen 7—8°C mit 17,66 g Benzolsulfonylchlorid umgesetzt und über Nacht nachreagieren gelassen. Man versetzt mit 80 ml Wasser stellt mit Salzäure auf pH 5 ein, wäscht 2 mal mit Wasser, trocknet über $Na_2SO_4$, filtriert und engt

am Rotationsverdampfer ein. Man erhält 34,2 g (87,8% d.Th.) eines klaren, rotbraunen Harzes mit einer Viskosität von 1,96 Pa.s.

| Elementaranalyse: | ber. für $C_{20}H_{21}NO_5S$ | gef.: |
|---|---|---|
| %C: | 62,00 | 62,14 |
| %H: | 5,46 | 5,60 |
| %N: | 3,62 | 3,60 |
| %S: | 8,27 | 7,95. |

IR-Spektrum ($cm^{-1}$): 575,4, 685,5 und 738,2 Aryl; 1195,5 und 1397 $—SO_2O—O—$; 1620 cycl. Doppelbindung; 1640 Allyldoppelbindung; 1742 Carbonyl.

Beispiel 3:

Allylbicyclo[2.2.1]hept-5-en-2,3-dicarbonsäure-N-(2'-benzolsulfonyloxyethyl)imid

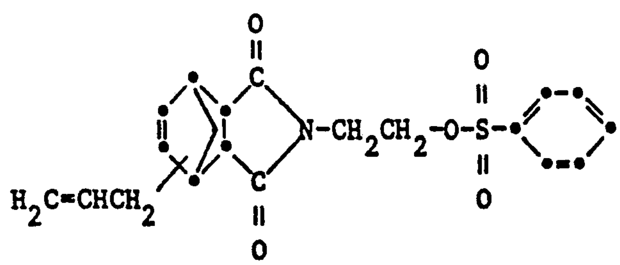

a) Allylbicyclo[2.2.1]hept-5-en-2,3-dicarbonsäurean-N-(2'-hydroxyethyl)imid

Man legt 760 g Allylbicyclo[2.2.1]hept-5-en-2,3-dicarbonsäureanhydrid vor und tropft unter Rühren 227,55 g Monoethanolamin ein. Man erhitzt während 2 Stunden auf Rückfluss und destilliert sodann Wasser und etwas überschüssiges Monoethanolamin ab, bis eine Temperatur von 175°C erreicht ist. Danach erniedrigt man den Druck auf 2670 Pa. Man kühlt auf 100°C ab und rektifiziert das Produkt bei 4,9 Pa. Zwischen 170 und 174°C gehen 712,4 g (77,3% d.Th.) eines blassgelben Oels mit folgenden Kenndaten über:

$$n_{25}^{D} = 1,5344$$
$$\eta\ 25 = 2,43 \text{ Pa.s}$$

| Analyse: | ber. | gef.: |
|---|---|---|
| %C: | 68,00 | 68,08 |
| %H: | 6,93 | 7,06 |
| %N: | 5,66 | 5,61 |

IR-Spektrum ($cm^{-1}$): 1619 cyclische Doppelbindung; 1641 Allylgruppe; 1697 Carbonylgruppe; 1768 Carbonyl im cyclischen Imid; 3449 Hydroxylgruppe.

b) Man setzt 123 g des gemäss (a) erhaltenen Produkts,
55,55 g Triethylamin in
400 g Toluol mit
88,31 g Benzolsulfonylchlorid
so um, wie dies in den vorhergehenden Beispielen beschrieben ist. Man erhält 171,25 g (88,5% d.Th.) eines rotbraunen, klaren Harzes mit einer Viskosität von 0,426 Pa.s bei 80°C.

| Elementaranalyse: | ber. für $C_{20}H_{21}NO_5S$ | gef.: |
|---|---|---|
| %C: | 60,00 | 62,29 |
| %H: | 5,46 | 5,56 |
| %N: | 3,62 | 3,48 |
| %S: | 8,27 | 8,13. |

Beispiel 4

Allylbicyclo[2.2.1]hept-5-en-2,3-dicarbonsäure-N-methansulfonyloxyimid

Man setzt 43,8 g des Produkts hergestellt gemäss Beispiel 1a,
22,2 g Triethylamin,
160 g Toluol und
22,91 g Methansulfonylchlorid

nach dem im Beispiel 1b beschriebenen Verfahren um und erhält 46,9 g (79% d.Th.) eines dunkelbraunen Harzes; $\eta_{80}$ = 0,31 Pa.s.

| Elementaranalyse: | ber. für $C_{13}H_{15}NO_5S$ | gef.: |
|---|---|---|
| %C: | 52,52 | 52,46 |
| %H: | 5,09 | 5,08 |
| %N: | 4,71 | 4,79 |
| %S: | 10,78 | 10,57 |

IR-Spektrum ($cm^{-1}$): 1190 und 1388 —$SO_2$—O—; 1739 und 1782 Carbonyl.

Beispiel 5:

Allylbicyclo[2.2.1]hept-5-en-2,3-dicarbonsäure-N-(2',2'-dimethyl-3'-methanesulfonyloxypropyl)imid

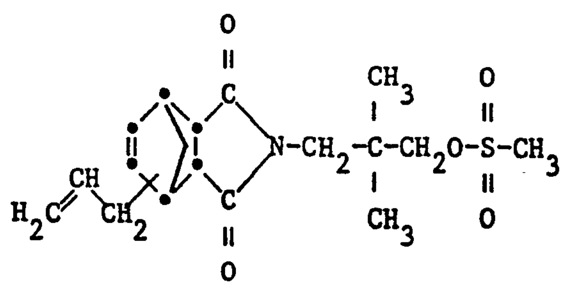

a) Allylbicyclo[2.2.1]hept-5-en-2,3-dicarbonsäure-N-(2',2'-dimethyl-3'-hydroxypropyl)imid

Man setzt 204 g Allylbicyclo[2.2.1]hept-5-en-2,3-dicarbonsäureanhydrid mit 103 g Neopentanolamin während 4 Stunden bei 120°C um, und destilliert das Produkt. Man erhält zwischen 169 und 172°C bei 2,53 Pa 268 g (92,7% d.Th.) eines gelben Oels mit einer Brechungszahl $n_{25}^{D}$ von 1,5190 und einer Viskosität von 6,21 Pa.s.

| Analyse: | ber.: | gef.: |
|---|---|---|
| %C: | 70,56 | 70,67 |
| %H: | 8,01 | 8,13 |
| %N: | 4,84 | 4,77 |
| %OH: | 5,88 | 5,69 |

Der Hydroxylgruppengehalt wird durch Acetylierung mit Essigsäureanhydrid in Pyridin bestimmt.

b) Man setz 72,25 g des gemäss (a) hergestellen Produkts,

27,32 g Triethylamin,

250 g Toluol und

28,64 g Methansulfonylchlorid

nach dem im Beispiel 1b beschriebenen Verfahren um und erhält 81,75 g (89,1% d.Th.) eines braunen klaren Harzes, $\eta_{80}$ = 0,414 Pa.s.

| Elementaranalyse: | ber. für $C_{18}H_{25}NO_5S$ | gef.: |
|---|---|---|
| %C: | 58,84 | 59,89 |
| %H: | 6,86 | 6,99 |
| %N: | 3,81 | 3,77 |
| %S: | 8,72 | 8,02. |

Beispiel 6

Allylbicyclo[2.2.1]hept-5-en-2,3-dicarbonsäure-N-[2'-(2''-p-toluolsulfonyloxyethoxy)-ethyl]imid

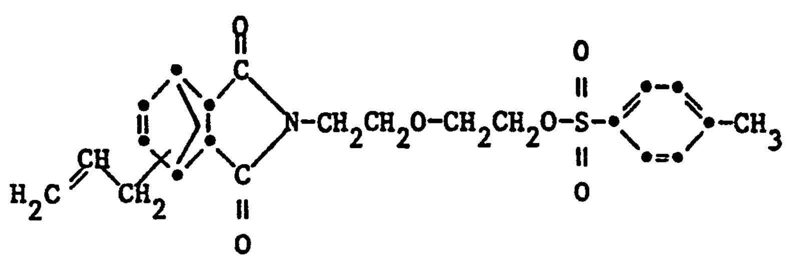

a) Allylbicyclo[2.2.1]hept-5-en-2,3-dicarbonsäure-N-[2'-(2''-hydroxyethoxy)ethyl]imid

Man setzt 120 g Allylbicyclo[2.2.1]hept-5-en-2,3-dicarbonsäureanhydrid mit 67,9 g Diglykolamin bei 140°C um, erhitzt auf 200°C und erniedrigt den Druck auf 10,6 Pa. Man erhält 142,6 g eines gelben Oels mit einer Viskosität von 1,55 Pa.s bie 25°C und einer $n_{25}^{D}$ von 1,5230.

| Analyse: | ber.: | gef.: |
|---|---|---|
| %C: | 65,96 | 65,74 |
| %H: | 7,27 | 7,39 |
| %N: | 4,81 | 4,71 |
| %OH: | 5,84 | 5,34 |

b) Man setzt 72,75 g des gemäss (a) hergestellten Produkts,
27,9 g Triethylamin,
250 g Toluol und
47,6 g p-Toluolsulfonylchlorid
nach dem im Beispiel 1b beschriebenen Verfahren um und erhält 93,1 g (83,7% d.Th.) eines rotbraunen flüssigen Harzes mit einer Viskosität von 3,1 Pa.s bei 40°C.

| Elementaranalyse: | ber. für $C_{23}H_{27}NO_5S$ | gef.: |
|---|---|---|
| %C: | 62,01 | 62,01 |
| %H: | 6,11 | 6,17 |
| %N: | 3,14 | 3,06 |
| %S: | 7,20 | 6,80. |

Beispiel 7

Allylbicyclo[2.2.1]hept-5-en-2,3-dicarbonsäure-N-[2'-(2''-naphthalinsulfonyloxy)ethyl]imid

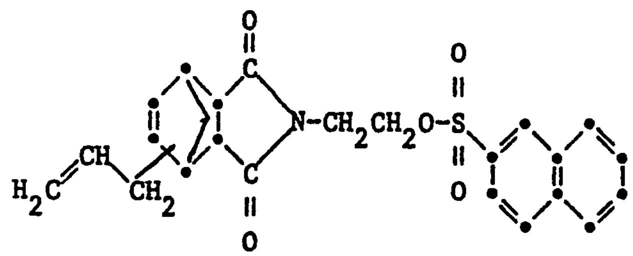

Man streut 90,67 g fein pulverisiertes Naphthalin-2-sulfochlorid unter starkem Rühren in eine Lösung von 98,8 g Hydroxyethylimid gemäss Beispiel 3a und 44,52 g Triethylamin in 500 g Toluol bei einer Temperatur von 0—5°C. Man lässt das Reaktionsgemisch sich auf Raumtemperatur erwärmen, rührt einige Studen und neutralisiert dann mit 1N-Salzsäure. Man wäscht 3 mal mit 250 ml $H_2O$, trocknet über 30 g $Na_2SO_4$, filtriert und destilliert das Toluol am Rotationsverdampfer ab (130°C/15 mm Hg). Man erhält 150,8 g (81,8% d.Th.) einer braunen Flüssigkeit, die beim Abkühlen zu einer wachsartigen Masse erstart, welche zwischen 66 und 74°C schmilzt.

| Elementaranalyse: | ber. für $C_{24}H_{23}NO_5S$ | gef.: |
|---|---|---|
| %C: | 65,90 | 65,51 |
| %H: | 5,26 | 5,40 |
| %N: | 3,20 | 3,28 |
| %S: | 7,32 | 7,08. |

Beispiel 8

Allylbicyclo[2.2.1]hept-5-en-2,3-dicarbonsäure-N-(4'-methansulfonyloxyphenyl)imid

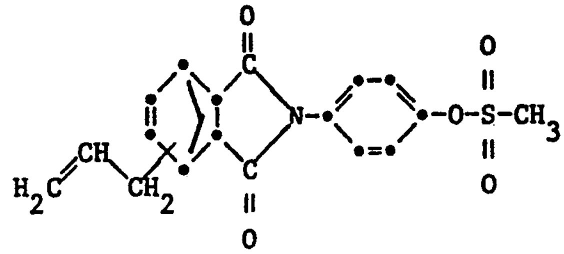

a) Allylbicyclo[2.2.1]hept-5-en-2,3-dicarbonsäure-N-(4'-hydroxyphenyl)imid

Man erhitzt eine Lösung von 101 g Allylbicyclo[2.2.1]hept-5-en-2,3-dicarbonsäureanhydrid und 54,5 g 4-Aminophenol in 155 g Toluol zum Sieden, und trennt das bei der Imidisierung gebildete Wasser in einem Hahnschen Aufsatz ab.

b) Diese Lösung verdünnt man mit weiteren 350 g Toluol, setzt 55,6 g Triethylamin zu, kühlt auf 0°C ab, und tropft unter Rühren 57,05 g Methansulfochlorid ein, wobei die Temperatur durch äussere Kühlung zwischen 5 und 8°C gehalten wird. Danach rührt man noch über Nacht bei Zimmertemperatur. Man stellt mit 1N HCl auf pH = 5,5, und wäscht mit Wasser, trocknet und isoliert das Produkt, wie im Beispiel 7 beschrieben. Ausbeute: 162 g entsprechend 86,86% d.Th.

| Elementaranalyse: | ber. für $C_{19}H_{19}NO_5S$ | gef.: |
|---|---|---|
| %C: | 61,11 | 61,66 |
| %H: | 5,13 | 5,24 |
| %N: | 3,75 | 3,70 |
| %S: | 8,59 | 8,37. |
| Molgewicht | 373,4 | 381 |

Beispiel 9

Allylbicyclo[2.2.1]hept-5-en-2,3-dicarbonsäure-N-(2'-methansulfonyloxyethyl)imid

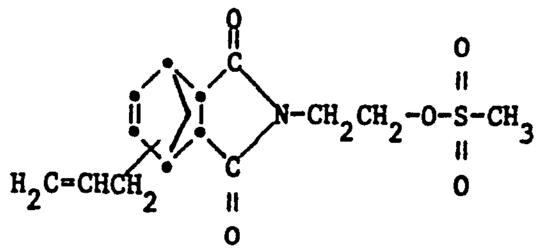

49,26 g Allylbicyclo[2.2.1]hept-5-en-2,3-dicarbonsäure-N-(2'-hydroxyethyl)imid (hergestellt gemäss Beispiel 3a) und 22,26 g Triethylamin werden in 160 g Toluol gelöst, und die Lösung wird unter Stickstoff auf 5°C gekühlt. Man tropft untere Rühren während 1 Stunde 22,91 g Methansulfochlorid zu, wobei die Temperatur durch äussere Kühlung zwischen 5 und 10°C gehalten wird. Danach rührt man noch während 18 Stunden bei Raumtemperatur. Man stellt mit 1N HCl auf pH 5 ein, wäscht mit Wasser, trocknet und isoliert das Produkt, wie im Beispiel 7 beschrieben. Mann erhält 54 g (83,0% d.Th), $\eta_{40°C}$ = 2138,4 mPa.s, $n^D_{25}$ = 1,5238.

| Elementaranalyse: | ber. für $C_{15}H_{19}NO_5S$ | gef.: |
|---|---|---|
| %C: | 55,37 | 55,71 |
| %H: | 5,89 | 6,00 |
| %N: | 4,30 | 4,28 |
| %S: | 9,85 | 9,47. |

Anwendungsbeispiele

Die unten angegebenen Harz A und B werden in Gegenwart von N-Sulfonyloxy-Imiden gemäss der vorliegenden Erfindung, die als latente Katalysatoren wirken, gehärtet.

Harz A: N,N'-Hexamethylen-bis(allylbicyclo[2.2.1]hept-5-en-2,3-dicarbonsäureimid, hergestellt gemäss Beispiel 9 der EP—A 0 105 024.

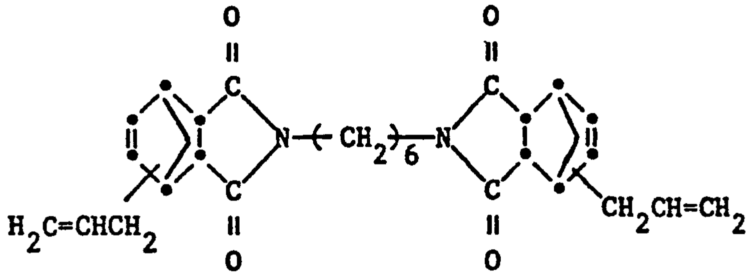

Harz B: Bis[4-(allylbicyclo[2.2.1]hept-5-en-2,3-dicarbonsäureimidophenyl)methan], hergestellt gemäss Beispiel 11 der EP—A—0 105 024.

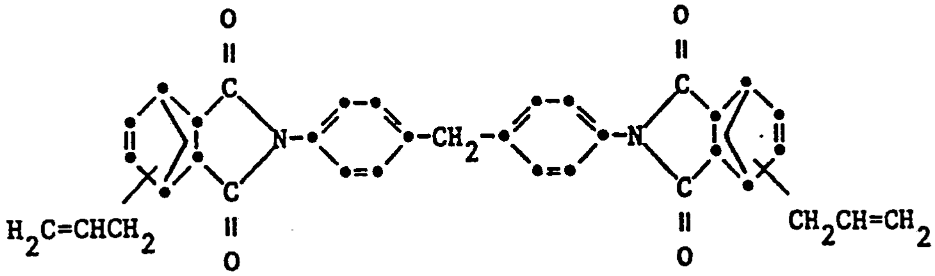

Das Harz A resp. B wird jeweils mit 1% des Katalysators versetzt, erhitzt, evakuiert, in Strahlformen von 150 × 150 × 4 $mm^3$ gegossen und während 2 Stunden bei 190°C (4 Stunden in den Beispielen XII und XIII) und 2 Stunden bei 250°C gehärtet. Die Eigenschaften der gehärteten Produkte sind in der Tabelle 1 angegeben.

Tabelle 1:

| Anw. Beisp.Nr. | Harz | Katalysator gemäss Beisp.Nr. | Biegefestigkeit $N/mm^2$ (DIN 53452) | Randfaserdehnung % (DIN 53452) | Schlagbiegefestigkeit $kJ/m^2$ (DIN 53453) | $T_G$ * °C |
|---|---|---|---|---|---|---|
| I | A | 1 | 112,2 | 4,3 | 11,3 | 196 |
| II | B | 1 | 99,0 | 3,4 | 6,1 | 280 |
| III | A | 2 | 95,6 | 3,4 | 11,4 | 193 |
| IV | B | 2 | 20,5 | 0,6 | 0,9 | 275 |
| V | B | 3 | 52,8 | 1,7 | 3,2 | 289 |
| VI | A | 4 | 117,6 | 5,2 | 16,4 | 251 |
| VII | B | 4 | 82,5 | 2,8 | 6,6 | 266 |
| VIII | B | 5 | 80,0 | 2,8 | 7,5 | 302 |
| IX | A | 6 | 111,1 | 4,4 | 10,5 | 186 |
| X | B | 6 | 60,1 | 2,0 | 2,9 | 289 |
| XI | A | 7 | 104,0 | 4,3 | 13,4 | 195 |
| XII | B | 8 | 65,7 | 2,0 | 4,7 | 281 |
| XIII | B | 9 | 88,7 | 2,9 | 7,9 | 300 |

* Bestimmt mit einem Thermoanalyser TA 2000 der Fa.Mettler (Greifensee, CH).

**Patentansprüche**

1. Imide der Formel I

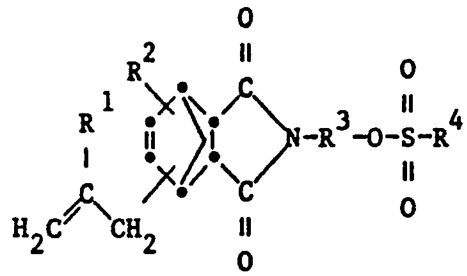

(I),

worin $R^1$ und $R^2$ unabhängig voneinander Wasserstoff oder Methyl bedeuten, $R^3$ eine direkte Bindung oder ein $C_2$—$C_{20}$-aliphatischer, ein durch O-Atome in der Kette unterbrochener $C_2$—$C_{20}$-aliphatischer, ein ein- oder mehr-kerniger $C_5$—$C_{20}$-cycloaliphatischer oder ein $C_6$—$C_{20}$-aromatischer Rest ist oder für eine Gruppe der Formel II steht

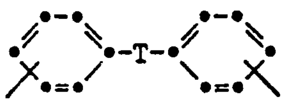

(II),

worin T Methylen, Isopropyliden, CO, O, S oder $SO_2$ bedeutet, und $R^4$ für $C_1$—$C_{12}$-Alkyl, $C_5$—$C_8$-Cycloalkyl, $C_6$—$C_{10}$-Aryl, Benzyl oder $C_7$—$C_{12}$-Alkaryl steht.

2. Imide der Formel I nach Anspruch 1, worin $R^1$ und $R^2$ je Wasserstoff bedeuten.

3. Imide der Formel I nach Anspruch 1, worin $R^3$ eine direkte Bindung, ein $C_2$—$C_{10}$-aliphatischer Rest oder eine Gruppe $—CH_2CH_2-(OCH_2CH_2)_m-$ mit m = 1 oder 2, ein $C_5$—$C_6$-cycloaliphatischer oder ein $C_6$—$C_{10}$-aromatischer Rest ist oder für eine Gruppe der Formel II nach Anspruch 1 steht, worin T Methylen oder Isopropyliden bedeutet.

4. Imide der Formel I nach Anspruch 1, worin $R^4$ für $C_1$—$C_6$-Alkyl, $C_5$—$C_6$-Cycloalkyl, $C_6$—$C_{10}$-Aryl oder $C_7$—$C_{12}$-Alkaryl steht.

5. Imide der Formel I nach Anspruch 1, worin $R^3$ eine direkte Bindung oder den Rest

$-CH_2CH_2-$,

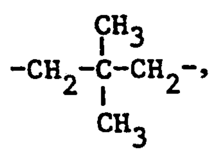 $-CH_2CH_2OCH_2CH_2-$ oder 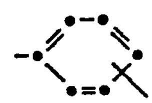 und

$R^4$ Methyl, Phenyl, 2-Naphthyl oder p-Tolyl bedeuten.

6. Imide der Formel I nach Anspruch 1, worin $R^1$ und $R^2$ je Wasserstoff bedeuten, $R^3$ für eine direkte Bindung oder für den Rest $—CH_2CH_2—$ und $R^4$ für Methyl oder Phenyl stehen.

7. Verfahren zur Herstellung von Imiden der Formel I nach Anspruch 1, dadurch gekennzeichnet, dass man ein Anhydrid der Formel III

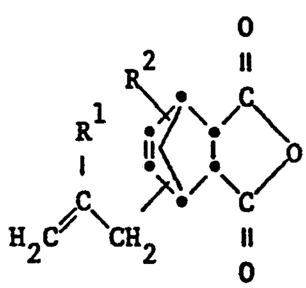

(III)

bei erhöhter Temperatur und unter Abdestillieren des bei der Reaktion entstehenden Wassers mit einer Verbindung der Formel IV

$$H_2N—R^3OH \quad (IV)$$

zu einer Verbindung der Formel V umsetzt,

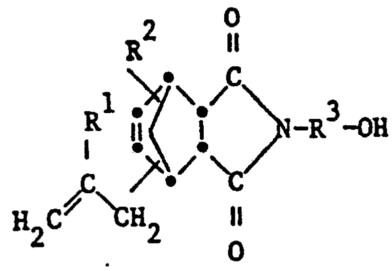

(V)

und diese anschliessend unter Kühlung und in Anwesenheit eines HCl-Akzeptors mit einem Sulfonsäurechlorid der formel VI umsetzt,

$$R^4SO_2Cl \qquad (VI)$$

wobei $R^1$, $R^2$, $R^3$ und $R^4$ die im Anspruch 1 angegebene Bedeutung haben.

8. Härtbare Gemische enthaltend
(a) ein oder mehrere Imide der Formel I nach Anspruch 1 und
(b) ein Alkenylnadicimid.

**Revendications**

1. Imides répondant à la formule I:

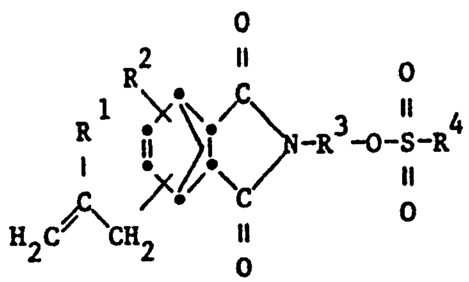

(I),

dans laquelle

$R^1$ et $R^2$ représentent chacun, indépendamment l'un de l'autre, l'hydrogène ou un méthyle,

$R^3$ représente un liaison directe, un radical aliphatique en $C_2$—$C_{20}$ dont la chaîne est éventuellement interrompue par des atomes d'oxygène, un radical cycloaliphatique en $C_5$—$C_{20}$ à un ou à plusieurs noyaux, un radical aromatique en $C_6$—$C_{20}$ ou un radical répondant à le formule II:

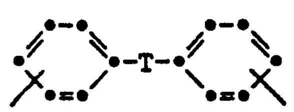

(II),

dans laquelle T représente un méthylène, un isopropylidène, CO, O, S ou $SO_2$ et,

$R^4$ représente un alkyle en $C_1$—$C_{12}$, un cycloalkyle en $C_5$—$C_8$, un aryle en $C_6$—$C_{10}$, un benzyle ou un alkilaryle en $C_7$—$C_{12}$.

2. Imides de formule I selon la revendication 1, dans lesquels $R^1$ et $R^2$ représentent chacun l'hydrogène.

3. Imides de formule I selon la revendication 1, dans lesquels $R^3$ représente un liaison directe, un radical aliphatique en $C_2$—$C_{10}$, un radical $-CH_2CH_2-[-OCH_2CH_2]_m-$ dans lequel m est égal à 1 ou à 2, un radical cycloalihatique en $C_5$ ou $C_6$, un radical aromatique en $C_6$—$C_{10}$ ou un radical de formule II selon la revendication 1 dans lequel T représente un radical méthylène ou isopropylidène.

4. Imides de formule I selon la revendication 1, dans lesquels $R^4$ représente un alkyle en $C_1$—$C_6$, un cycloalkyle en $C_5$ ou $C_6$, un aryle en $C_6$—$C_{10}$ ou un alkylaryle en $C_7$—$C_{12}$.

5. Imides de formule I selon la revendication 1, dans lesquels $R^3$ représente un liaison directe ou un radical:

$-CH_2CH_2-$, 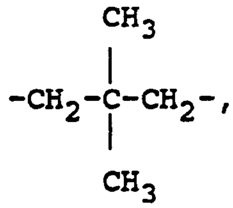, $-CH_2CH_2OCH_2CH_2-$ ou 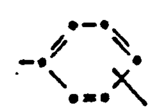

et $R^4$ représente un radical méthyle, phényle, naphtyle-2 ou p-tolyle.

6. Imides de formule I selon la revendiction 1, dans lesquels $R^1$ et $R^2$ représentent chacun l'hydrogène, $R^3$ représente une liaison directe ou un radical $—CH_2CH_2—$ et $R^4$ un radical méthyle ou phényle.

7. Procédé pour préparer des imides de formule I selon la revendication 1, procédé caractérisé en ce qu'on fait réagir un anhydride de formule III:

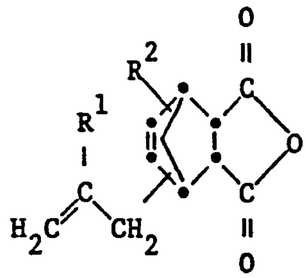

(III)

à température élevée et tout en chassant par distillation l'eau engendrée par la réaction, avec un composé de formule IV:

$$H_2N—R^3OH \qquad (IV),$$

de mainère à obtenir un composé de formule V:

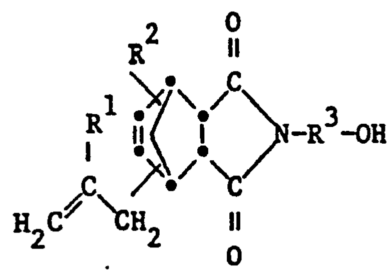

(V)

puis on fait réagir celui-ci, tout en refroidissant et en opérant en présence d'un accepteur d'HCl, avec un chlorure d'acide sulfonqiue de formule VI:

$$R^4SO_2Cl \qquad (VI),$$

formules dans lesquelles $R^1$, $R^2$, $R^3$ et $R^4$ ont les significations qui leur ont été données à la revendication 1.

8. Mélanges durcissables qui contiennent:
(a) un ou plusieurs imides de Formule I selon la revendication 1 et
(b) un alcényl-nadicimide.

**Claims**

1. An imide of formula I

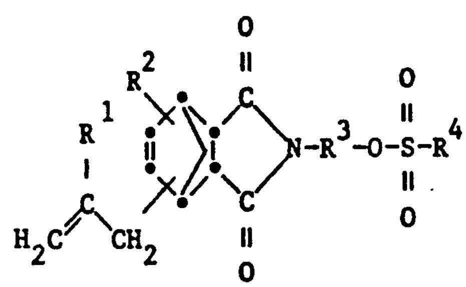

(I),

wherein each of $R^1$ and $R^2$ independently of the other is hydrogen or methyl, $R^3$ is a direct bond or a $C_2—C_{20}$ aliphatic radical or a $C_2—C_{20}$ aliphatic radical which is interrupted in the chain by oxygen atomes, or is a mono- or polynuclear $C_5—C_{20}$ cycloaliphatic radical, a $C_6—C_{20}$ aromatic radical or a group of formula II

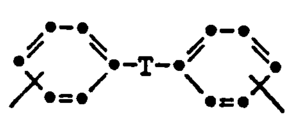

(II),

wherein T is methylene, isopropylidene, CO, O, S or $SO_2$, and $R^4$ is $C_1$—$C_{12}$ alkyl, $C_5$—$C_8$ cycloalkyl, $C_6$—$C_{10}$ aryl, benzyl or $C_7$—$C_{12}$ alkaryl.

2. An imide of formula I according to claim 1, wherein each of $R^1$ and $R^2$ is hydrogen.

3. An imide of formula I according to claim 1, wherein $R^3$ is a direct bond, a $C_2$—$C_{10}$ aliphatic radical or a $-CH_2CH_2-[OCH_2CH_2]_m-$ group, where m is 1 or 2, a $C_5$—$C_6$ cycloaliphatic or a $C_6$—$C_{10}$ aromatic radical or a group of formula II according to claim 1, wherein T is methylene or isopropylidene.

4. An imide of formula I according to claim 1, wherein $R^4$ is $C_1$—$C_6$ alkyl, $C_5$—$C_6$ cycloalkyl, $C_6$—$C_{10}$ aryl or $C_7$—$C_{12}$ alkaryl.

5. An imide of formula I according to claim 1, wherein $R^3$ is a direct bond or the radical,

$-CH_2CH_2-$,

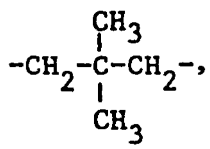,

$-CH_2CH_2OCH_2CH_2-$ or

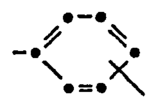

$R^4$ is methyl, phenyl, 2-naphthyl or p-tolyl.

6. An imide of formula I according to claim 1, wherein each of $R^1$ and $R^2$ is hydrogen, $R^3$ is a direct bond or the radical $-CH_2CH_2-$ and $R^4$ is methyl or phenyl.

7. A process for the preparation of an imide of formula I according to claim 1, which process comprises reacting an anhydride of formula III

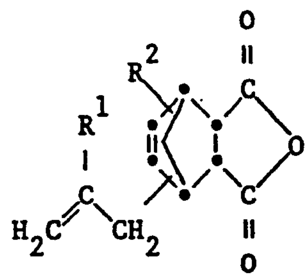

(III)

with a compound of formula IV

$$H_2N-R^3OH \qquad (IV)$$

at elevated temperature and with removal by distillation of the water forming during the reaction, to give a compound of formula V

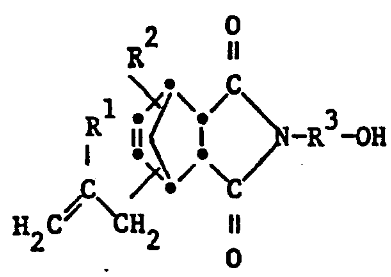

(V)

and subsequently reacting this compound with a sulfonyl chloride of formula VI

$$R^4SO_2Cl \qquad (VI)$$

with cooling and in the presence of an HCl acceptor, $R^1$, $R^2$, $R^3$ and $R^4$ being as defined in claim 1.

8. A curable mixture containing

(a) one or more imides of formula I according to claim 1 and

(b) an alkenylnadicimide.